Europäisches Patentamt

European Patent Office

Office européen des brevets

(19)

(11) **EP 1 316 304 A1**

(12) **DEMANDE DE BREVET EUROPEEN**

(43) Date de publication:
**04.06.2003 Bulletin 2003/23**

(51) Int Cl.⁷: **A61K 7/48**

(21) Numéro de dépôt: **02292810.5**

(22) Date de dépôt: **12.11.2002**

(84) Etats contractants désignés:
**AT BE BG CH CY CZ DE DK EE ES FI FR GB GR IE IT LI LU MC NL PT SE SK TR**
Etats d'extension désignés:
**AL LT LV MK RO SI**

(30) Priorité: **28.11.2001 FR 0115375**

(71) Demandeur: **L'OREAL**
**75008 Paris (FR)**

(72) Inventeurs:
- **Biatry, Bruno**
  **94300 Vincennes (FR)**
- **Lheureux, Eric**
  **91230 Montgeron (FR)**

(74) Mandataire: **Rasson, Catherine**
**L'OREAL-DPI**
**6 rue Bertrand Sincholle**
**92585 Clichy Cedex (FR)**

(54) **Utilisations cosmétiques et/ou dermatologiques d'une composition contenant au moins un actif hydrophile sensible à l'oxydation stabilisé par au moins un polymère ou copolymère de N-vinylimidazole**

(57) L'invention se rapporte à l'utilisation cosmétique et/ou dermatologique d'une composition contenant, dans un milieu physiologiquement acceptable comprenant une phase aqueuse, au moins un actif hydrophile sensible à l'oxydation choisi parmi l'acide ascorbique et ses dérivés et au moins un polymère ou copolymère de N-vinylimidazole non réticulé, ledit actif et ledit polymère ou copolymère étant tous deux dans la phase aqueuse, pour favoriser la synthèse des céramides épidermiques, et/ou pour améliorer la fonction barrière de la peau.

L'invention concerne également un procédé cosmétique d'hydratation de la peau comprenant l'application sur la peau d'une composition telle que définie précédemment.

EP 1 316 304 A1

**Description**

La présente invention se rapporte à l'utilisation cosmétique et/ou dermatologique d'une composition contenant au moins un actif hydrophile sensible à l'oxydation et au moins un polymère ou copolymère de N-vinylimidazole , dans un milieu physiologiquement acceptable comprenant une phase aqueuse.

Il est connu d'introduire dans des compositions cosmétiques divers actifs destinés à apporter des traitements spécifiques à la peau et/ou aux cheveux. Toutefois, certains de ces actifs présentent l'inconvénient d'être instables en milieu aqueux et de se dégrader facilement au contact de l'eau, en particulier à cause de phénomènes d'oxydation. Ils perdent ainsi rapidement leur activité au cours du temps et cette instabilité va à l'encontre de l'efficacité recherchée.

On cherche ainsi depuis longtemps à formuler l'acide ascorbique ou vitamine C, du fait de ses nombreuses propriétés bénéfiques. En particulier, l'acide ascorbique stimule la synthèse du tissu conjonctif et notamment du collagène, renforce les défenses du tissu cutané contre les agressions extérieures telles que les rayonnements ultraviolets et la pollution, compense la déficience en vitamine E de la peau, dépigmente la peau et possède une fonction anti-radicaux libres. Ces deux dernières propriétés en font un excellent candidat comme actif cosmétique ou dermatologique pour lutter contre le vieillissement de la peau ou prévenir celui-ci. Malheureusement, en raison de sa structure chimique (d'alpha-cétolactone), l'acide ascorbique est très sensible à certains paramètres de l'environnement et notamment aux phénomènes d'oxydation. Il s'ensuit donc une dégradation rapide de l'acide ascorbique formulé en présence de ces paramètres, et plus particulièrement en présence d'oxygène, de lumière, d'ions métalliques, en fonction de la température, ou encore dans certaines conditions de pH (Pharm. Acta. Helv., 1969, 44, 611-667 ; STP Pharma, 1985, 4, 281-286).

Plusieurs solutions ont donc été envisagées dans l'art antérieur pour diminuer et/ou retarder la dégradation de l'acide ascorbique.

Il a ainsi été proposé d'utiliser l'acide ascorbique sous forme de dérivé chimique (ascorbyl phosphate de magnésium, ou esters d'acides gras et d'acide ascorbique), mais la biodisponibilité de ces dérivés est très faible (J. Am. Acad. Dermatol., 1996, 34, 29-33).

L'instabilité de l'acide ascorbique vis à vis de l'oxygène a pu être améliorée en utilisant des conditionnements particuliers comme des bi-compartiments sous atmosphère inerte tels que décrits dans le brevet US-5,935,584, ou bien encore par l'utilisation d'émulsions à deux phases dont l'une est constituée d'une poudre sèche contenant l'acide ascorbique et la seconde d'une phase liquide. Le mélange des deux phases doit s'effectuer au moment de l'utilisation (WO98/43598). Ces solutions présentent des inconvénients au niveau du coût et de la complexité des fabrications ainsi que des contraintes importantes au niveau de l'utilisation.

Une autre solution proposée dans l'art antérieur consiste à utiliser des glycols ou des polyols en forte concentration afin de diminuer la solubilité de l'oxygène dans la formulation, protégeant ainsi l'acide ascorbique (WO-96/24325, EP 0 755 674, US-5,981,578). Les polyols peuvent éventuellement être incorporés dans des liposomes comme décrit dans le brevet US-6,020,367. Mais ces solutions présentent l'inconvénient de conduire à des formulations collantes dont la cosméticité est difficile à améliorer. Par ailleurs la présence d'une forte concentration de ces composés peut provoquer des phénomènes d'irritation.

L'acide ascorbique peut également être formulé dans des milieux anhydres tels que les silicones (US-6,194,452) qui sont capables de créer une barrière anhydre autour de l'acide ascorbique. Un inconvénient majeur de telles solutions résulte du manque de fraîcheur à l'application.

Il subsiste donc le besoin d'une composition utilisable notamment dans le domaine cosmétique, dans laquelle un actif hydrophile et instable en milieu oxydant est stabilisé, qui soit confortable lors de l'application, qui ne provoque aucune irritation de la peau après application, et qui soit compatible avec les contraintes d'une mise en oeuvre industrielle de son procédé de fabrication.

L'acide ascorbique est capable d'améliorer le profil lipidique des épidermes reconstruits en modifiant la lipogénèse, et provoque en particulier une augmentation de la synthèse des céramides (J. Invest. Dermatol., 109, 1997, p. 348-355). Parallèlement, cet effet a également été mis en évidence pour les dérivés de l'acide ascorbique tels que l'ascorbyl phosphate de magnésium ou l'ascorbyl glucoside. Des études ont aussi montré que la fonction barrière des épidermes reconstruits était améliorée après traitement par l'acide ascorbique (EP-1 145 706, EP-1 145 710).

On comprend alors l'intérêt d'utiliser ces dérivés afin de maintenir et de renforcer l'intégrité des lipides de la peau, et ainsi de palier les problèmes d'hydratation.

En complément de cette activité sur la synthèse des lipides, un effet prodifférenciant sur les kératinocytes de l'acide ascorbique a été récemment mis en évidence (Histochem. Cell. Biol., 116(6), 2001, p. 287-297). Cet effet a pour conséquence de palier l'altération de ce mécanisme de différenciation des kératinocytes qui conduit à une accumulation des squames à la surface de la peau. Ce phénomène modifie les interactions entre la surface de la peau et la lumière et est responsable d'un aspect terne et rugueux de la peau.

L'acide ascorbique et ses dérivés pourront donc avantageusement être utilisés pour lutter contre le teint terne et pour maintenir l'éclat de la peau.

Le but de la présente invention est de proposer une composition contenant un actif sensible à l'oxydation choisi parmi l'acide ascorbique et ses dérivés, présentant de bonnes propriétés cosmétiques, tant au niveau du toucher qu'au niveau de la tolérance, dont la conservation dans le temps ne demande pas de précautions particulières, et qui conserve l'activité dudit actif dans l'amélioration de la synthèse des céramides et de la fonction barrière de la peau, ainsi que dans l'amélioration de la différenciation des kératinocytes.

La Demanderesse a découvert, de manière fortuite, que l'utilisation de polymères ou copolymères de N-vinylimidazole non réticulés dans des compositions dont la phase aqueuse renferme un actif sensible à l'oxydation, tel que l'acide ascorbique ou un de ses dérivés, permettait d'atteindre le but précité.

Dans l'art antérieur, certains composés possédant une structure imidazole ont été décrits pour leurs propriétés stabilisatrices. Ainsi, dans la demande de brevet EP 0 586 106, plusieurs molécules à base d'imidazole sont utilisées pour stabiliser certains rétinoïdes contre les dégradations chimiques. D'autre part, des émulsifiants polymériques constitués de N-vinylimidazole, d'acrylates d'alkyles et de vinyl-acétates sont décrits dans le brevet US-4,057,622. Ils sont utilisés dans le but de remplacer les émulsifiants connus afin de pallier leurs inconvénients, en particulier au niveau de l'odeur, et pour stabiliser les émulsions eau-dans-huile. Enfin des copolymères de N-vinylimidazole / N-vinylcaprolactam / N-vinylpyrrolidone sont décrits dans le brevet US-6,191,188. Ils entrent dans la fabrication de compositions renforçantes pour les cheveux.

A la connaissance de la demanderesse, des polymères ou copolymères comportant des unités N-vinylimidazoles n'ont jamais été associés à des actifs hydrophiles sensibles aux dégradations par oxydation dans le but d'améliorer leur stabilité en milieu aqueux. Ceci est vrai en particulier dans le cas de l'acide ascorbique.

La présente invention a donc pour objet l'utilisation cosmétique et/ou dermatologique pour favoriser la synthèse des céramides et/ou améliorer la fonction barrière de la peau d'une composition, contenant, dans un milieu physiologiquement acceptable comprenant une phase aqueuse, au moins un actif hydrophile sensible à l'oxydation choisi parmi l'acide ascorbique et ses dérivés, et au moins un polymère ou copolymère de N-vinylimidazole non réticulé, ledit actif et ledit polymère ou copolymère étant tous deux dans la phase aqueuse. Le copolymère est présent en quantité suffisante pour stabiliser ledit actif hydrophile sensible à l'oxydation.

La présente invention a également pour objet l'utilisation cosmétique et/ou dermatologique pour lutter contre la rugosité de la peau et/ou maintenir et/ou améliorer l'éclat du teint, d'une composition contenant, dans un milieu physiologiquement acceptable comprenant une phase aqueuse, au moins un actif hydrophile sensible à l'oxydation choisi parmi l'acide ascorbique et ses dérivés, et au moins un polymère ou copolymère de N-vinylimidazole non réticulé, ledit actif et ledit polymère ou copolymère étant tous deux dans la phase aqueuse

L'invention a aussi pour objet l'utilisation d'une association constituée d'au moins un actif hydrophile sensible à l'oxydation choisi parmi l'acide ascorbique et ses dérivés, et d'au moins un polymère ou copolymère de N-vinylimidazole non réticulé, dans la phase aqueuse d'une composition cosmétique, en tant qu'agent pour favoriser la synthèse des céramides et/ou améliorer la fonction barrière de la peau.

L'invention a en outre pour objet l'utilisation d'une association constituée d'au moins un actif hydrophile sensible à l'oxydation choisi parmi l'acide ascorbique et ses dérivés, et d'au moins un polymère ou copolymère de N-vinylimidazole non réticulé, dans la phase aqueuse d'une composition cosmétique, en tant qu'agent pour lutter contre la rugosité de la peau et/ou maintenir et/ou améliorer l'éclat du teint.

Selon l'invention, par actif hydrophile on entend un composé ayant une solubilité dans l'eau d'au moins 0,25 % à température ambiante (25°C).

Selon l'invention, par actif hydrophile *sensible à l'oxydation* on entend tout actif d'origine naturelle ou synthétique susceptible de subir une dégradation par un mécanisme d'oxydation. Ce phénomène d'oxydation peut avoir plusieurs causes, en particulier la présence d'oxygène, de lumière, d'ions métalliques, une température élevée, ou encore certaines conditions de pH.

On peut citer à titre d'exemple et de façon non limitative : l'acide ascorbique et ses dérivés tels que ses sels ou ses esters, et particulièrement le 5,6-di-O-diméthylsilylascorbate (vendu par la Sté Exsymol sous la référence PRO-AA), le sel de potassuim du dl-alpha-tocopheryl-dl-ascorbyl-phosphate (vendu par la Sté Senju Pharmaceutical sous la référence SEPIVITAL EPC), l'ascorbyl phosphate de magnésium, l'ascorbyl phosphate de sodium (vendu par la Sté Roche sous la référence Stay-C 50).

Dans un aspect particulièrement avantageux, l'actif hydrophile sensible à l'oxydation est l'acide ascorbique.

Selon l'invention, par polymère ou copolymère de N-vinylimidazole non réticulé on entend tout polymère comportant des unités N-vinylimidazole, et ne comportant pas d'agent réticulant. Des copolymères convenant à la mise en oeuvre de l'invention sont les copolymères associant des sous unités N-vinylimidazole avec des sous unités N-vinylpyrrolidone et/ou N-vinylcaprolactam.

Dans un aspect avantageux de l'invention, le copolymère possède une fraction molaire en unité N-vinylimidazole comprise entre 0,1 et 1, et plus préférentiellement entre 0,4 et 0,9.

Selon un aspect avantageux de l'invention le rapport molaire entre l'équivalent unité N-vinylimidazole et l'actif hydrophile sensible à l'oxydation varie entre 0,004 et 16 et préférentiellement entre 0,01 et 1.

De façon préférentielle on utilisera un copolymère de N-vinylimidazole / N-vinylpyrrolidone.

La masse molaire moyenne en poids des polymères de N-vinylimidazole sera avantageusement comprise entre 1000 et $1 \times 10^7$ et de préférence entre 5000 et $5 \times 10^6$.

On pourra utiliser à cet effet, le copolymère vinylpyrrolidone/vinylimidazole (50/50) ayant une masse molaire moyenne en poids de 1 200 000 vendu sous la référence LUVITEC VPI 55K72W par la société BASF ou le copolymère vinylpyrrolidone/vinylimidazole (50/50) ayant une masse molaire moyenne en poids de 10 000 vendu sous la référence LUVITEC VPI 55K18P par la société BASF.

Le copolymère est présent dans la composition selon l'invention en quantité suffisante pour obtenir l'effet recherché, c'est à dire en quantité suffisante pour stabiliser l'actif hydrophile sensible à l'oxydation. De préférence le copolymère est présent à une concentration comprise entre 0,1 et 5 % en poids, par rapport au poids total de la phase aqueuse, et plus particulièrement à une concentration comprise entre 0,1 et 2 % en poids, par rapport au poids total de la phase aqueuse.

Les compositions utilisées selon l'invention sont destinées à une application topique sur la peau et/ou ses phanères et contiennent donc un milieu physiologiquement acceptable, c'est-à-dire compatible avec les tissus cutanés tels que la peau, le cuir chevelu, les cils, les sourcils, les cheveux, les ongles et les muqueuses. Ce milieu physiologiquement acceptable comprend une phase aqueuse et éventuellement un solvant organique physiologiquement acceptable choisi par exemple parmi les alcools inférieurs comportant de 1 à 8 atomes de carbone et en particulier de 1 à 6 atomes de carbone, comme l'éthanol, l'isopropanol, le propanol, le butanol ; les polyéthylène glycols ayant de 6 à 80 unités oxyde d'éthylène ; les polyols comme le propylène glycol, l'isoprène glycol, le butylène glycol, la glycérine, le sorbitol.

Quand le milieu physiologiquement acceptable est un milieu aqueux, il a généralement un pH compatible avec la peau, allant de préférence de 3 à 9 et mieux de 3,5 à 7,5.

Les compositions selon l'invention peuvent se présenter sous toutes les formes galéniques classiquement utilisées pour une application topique et notamment sous forme de solutions aqueuses, hydroalcooliques, d'émulsions huile-dans-eau (H/E) ou eau-dans-huile (E/H) ou multiple (triple : E/H/E ou H/E/H), de gels aqueux, ou de dispersions d'une phase grasse dans une phase aqueuse à l'aide de sphérules, ces sphérules pouvant être des nanoparticules polymériques telles que les nanosphères et les nanocapsules, ou des vésicules lipidiques de type ionique et/ou non ionique (liposomes, niosomes, oléosomes). Ces compositions sont préparées selon les méthodes usuelles.

En outre, les compositions utilisées selon l'invention peuvent être plus ou moins fluides et avoir l'aspect d'une crème blanche ou colorée, d'une pommade, d'un lait, d'une lotion, d'un sérum, d'une pâte, d'une mousse. Elles peuvent être éventuellement appliquées sur la peau sous forme d'aérosol. Elles peuvent aussi se présenter sous forme solide, et par exemple sous forme de stick.

Quand la composition utilisée selon l'invention comporte une phase huileuse, celle-ci contient de préférence au moins une huile. Elle peut contenir en outre d'autres corps gras.

Comme huiles utilisables dans la composition de l'invention, on peut citer par exemple :

- les huiles hydrocarbonées d'origine animale, telles que le perhydrosqualène ;
- les huiles hydrocarbonées d'origine végétale, telles que les triglycérides liquides d'acides gras comportant de 4 à 10 atomes de carbone comme les triglycérides des acides heptanoïque ou octanoïque ou encore, par exemple les huiles de tournesol, de maïs, de soja, de courge, de pépins de raisin, de sésame, de noisette, d'abricot, de macadamia, d'arara, de ricin, d'avocat, les triglycérides des acides caprylique/caprique comme ceux vendus par la société Stearineries Dubois ou ceux vendus sous les dénominations Miglyol 810, 812 et 818 par la société Dynamit Nobel, l'huile de jojoba, l'huile de beurre de karité;
- les esters et les éthers de synthèse, notamment d'acides gras, comme les huiles de formules $R^1COOR^2$ et $R^1OR^2$ dans laquelle $R^1$ représente le reste d'un acide gras comportant de 8 à 29 atomes de carbone, et $R^2$ représente une chaîne hydrocarbonée, ramifiée ou non, contenant de 3 à 30 atomes de carbone, comme par exemple l'huile de Purcellin, l'isononanoate d'isononyle, le myristate d'isopropyle, le palmitate d'éthyl-2-hexyle, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéaryl-malate, le citrate de triisocétyle, les heptanoates, octanoates, décanoates d'alcools gras ; les esters de polyol, comme le dioctanoate de propylène glycol, le diheptanoate de néopentylglycol et le diisononanoate de diéthylèneglycol ; et les esters du pentaérythritol comme le tétraisostéarate de pentaérythrityle ;
- les hydrocarbures linéaires ou ramifiés, d'origine minérale ou synthétique, tels que les huiles de paraffine, volatiles ou non, et leurs dérivés, la vaseline, les polydécènes, le polyisobutène hydrogéné tel que l'huile de parléam ;
- les alcools gras ayant de 8 à 26 atomes de carbone, comme l'alcool cétylique, l'alcool stéarylique et leur mélange (alcool cétylstéarylique), l'octyldodécanol, le 2-butyloctanol, le 2-hexyldécanol, le 2-undécylpentadécanol, l'alcool oléique ou l'alcool linoléique ;
- les huiles fluorées partiellement hydrocarbonées et/ou siliconées comme celles décrites dans le document JP-A-2-295912 ;

- les huiles de silicone comme les polyméthylsiloxanes (PDMS) volatiles ou non à chaîne siliconée linéaire ou cyclique, liquides ou pâteux à température ambiante, notamment les cyclopolydiméthylsiloxanes (cyclométhicones) telles que la cyclohexasiloxane ; les polydiméthylsiloxanes comportant des groupements alkyle, alcoxy ou phényle, pendant ou en bout de chaîne siliconée, groupements ayant de 2 à 24 atomes de carbone ; les silicones phénylées comme les phényltriméthicones, les phényldiméthicones, les phényltriméthylsiloxydiphényl-siloxanes, les diphényl-diméthicones, les diphénylméthyldiphényl trisiloxanes, les 2-phényléthyltriméthyl-siloxysilicates, et les polyméthylphénylsiloxanes ;
- leurs mélanges.

On entend par « huile hydrocarbonée » dans la liste des huiles citées ci-dessus, toute huile comportant majoritairement des atomes de carbone et d'hydrogène, et éventuellement des groupements ester, éther, fluoré, acide carboxylique et/ou alcool.

Les autres corps gras pouvant être présents dans la phase huileuse sont par exemple les acides gras comportant de 8 à 30 atomes de carbone, comme l'acide stéarique, l'acide laurique, l'acide palmitique et l'acide oléique ; les cires comme la lanoline, la cire d'abeille, la cire de Carnauba ou de Candellila, les cires de paraffine, de lignite ou les cires microcristallines, la cérésine ou l'ozokérite, les cires synthétiques comme les cires de polyéthylène, les cires de Fischer-Tropsch ; les résines de silicone telles que la trifluorométhyl-C1-4-alkyldimethicone et la trifluoropropyldimethicone ; et les élastomères de silicone comme les produits commercialisés sous les dénominations « KSG » par la société Shin-Etsu, sous les dénominations « Trefil », « BY29 » ou « EPSX » par la société Dow Corning ou sous les dénominations « Gransil » par la société Grant Industries.

Ces corps gras peuvent être choisis de manière variée par l'homme du métier afin de préparer une composition ayant les propriétés, par exemple de consistance ou de texture, souhaitées.

Selon un mode particulier de réalisation de l'invention, la composition selon l'invention est une émulsion eau-dans-huile (E/H) ou huile-dans-eau (H/E). La proportion de la phase huileuse de l'émulsion peut aller de 5 à 80 % en poids, et de préférence de 5 à 50 % en poids par rapport au poids total de la composition.

Les émulsions contiennent généralement au moins un émulsionnant choisi parmi les émulsionnants amphotères, anioniques, cationiques ou non ioniques, utilisés seuls ou en mélange, et éventuellement un co-émulsionnant. Les émulsionnants sont choisis de manière appropriée suivant l'émulsion à obtenir (E/H ou H/E). L'émulsionnant et le co-émulsionnant sont généralement présents dans la composition, en une proportion allant de 0,3 à 30 % en poids, et de préférence de 0,5 à 20 % en poids par rapport au poids total de la composition.

Pour les émulsions E/H, on peut citer par exemple comme émulsionnants les dimethicone copolyols tels que le mélange de cyclomethicone et de dimethicone copolyol, vendu sous la dénomination « DC 5225 C » par la société Dow Corning, et les alkyl-dimethicone copolyols tels que le Laurylmethicone copolyol vendu sous la dénomination "Dow Corning 5200 Formulation Aid" par la société Dow Corning et le Cetyl dimethicone copolyol vendu sous la dénomination Abil EM 90$^R$ par la société Goldschmidt. On peut aussi utiliser comme tensioactif d'émulsions E/H, un organopolysiloxane solide élastomère réticulé comportant au moins un groupement oxyalkyléné, tel que ceux obtenus selon le mode opératoire des exemples 3, 4 et 8 du document US-A-5,412,004 et des exemples du document US-A-5,811,487, notamment le produit de l'exemple 3 (exemple de synthèse) du brevet US-A-5,412,004. et tel que celui commercialisé sous la référence KSG 21 par la société Shin Etsu. On peut également utiliser comme émulsionnant un oligomère ou un polymère dérivé de polyoléfine à terminaison succinique, celui-ci est de préférence une polyoléfine à terminaison succinique estérifiée ou amidifiée, ou un sel d'une telle polyoléfine, et en particulier du polyisobutylène à terminaison succinique estérifiée ou amidifiée tels que les produits commercialisés sous les dénominations L5603 et L2721 et OS131769 par la société Lubrizol.

Pour les émulsions H/E, on peut citer par exemple comme émulsionnants, les émulsionnants non ioniques tels que les esters d'acides gras et de glycérol oxyalkylénés (plus particulièrement polyoxyéthylénés) ; les esters d'acides gras et de sorbitan oxyalkylénés ; les esters d'acides gras oxyalkylénés (oxyéthylénés et/ou oxypropylénés) ; les éthers d'alcools gras oxyalkylénés (oxyéthylénés et/ou oxypropylénés) ; les esters de sucres comme le stéarate de sucrose ; et leurs mélanges tels que le mélange de stéarate de glycéryle et de stéarate de PEG-40.

De façon connue, la composition cosmétique ou dermatologique de l'invention peut contenir également des adjuvants habituels dans le domaine cosmétique ou dermatologique, tels que les gélifiants hydrophiles ou lipophiles, les conservateurs, les solvants, les parfums, les charges, les filtres UV, les bactéricides, les absorbeurs d'odeur, les matières colorantes, les extraits végétaux, les sels. Les quantités de ces différents adjuvants sont celles classiquement utilisées dans le domaine considéré, et par exemple de 0,01 à 20 % du poids total de la composition. Ces adjuvants, selon leur nature, peuvent être introduits dans la phase grasse, dans la phase aqueuse et/ou dans les sphérules lipidiques.

Comme charges qui peuvent être utilisées dans la composition de l'invention, on peut citer par exemple les pigments, la poudre de silice ; le talc ; les particules de polyamide et notamment celles vendues sous la dénomination ORGASOL par la société Atochem ; les poudres de polyéthylène ; les microsphères à base de copolymères acryliques, telles que celles en copolymère diméthacrylate d'éthylène glycol/ methacrylate de lauryle vendues par la société Dow Corning sous la dénomination de POLYTRAP ; les poudres expansées telles que les microsphères creuses et notamment, les microsphères commercialisées sous la dénomination EXPANCEL par la société Kemanord Plast ou sous la dénomination MICROPEARL F 80 ED par la société Matsumoto ; les microbilles de résine de silicone telles que celles commercialisées sous la dénomination TOSPEARL par la société Toshiba Silicone ; et leurs mélanges. Ces charges peuvent être présentes dans des quantités allant de 0 à 20 % en poids et de préférence de 1 à 10 % en poids par rapport au poids total de la composition.

Selon un mode préféré de réalisation, les compositions conformes à l'invention peuvent comporter en plus au moins un agent photoprotecteur organique et/ou au moins un agent photoprotecteur inorganique actif dans l'UVA et/ou l'UVB (absorbeurs), hydrosolubles ou liposolubles ou bien insolubles dans les solvants cosmétiques couramment utilisés.

Les agents photoprotecteurs organiques sont notamment choisis parmi les anthranilates ; les dérivés cinnamiques ; les dérivés de dibenzoylméthane ; les dérivés salicyliques, les dérivés du camphre ; les dérivés de triazine tels que ceux décrits dans les demandes de brevet US 4367390, EP863145, EP517104, EP570838, EP796851, EP775698, EP878469, EP933376, EP507691, EP507692, EP790243, EP944624 ; les dérivés de la benzophénone ; les dérivés de β,β-diphénylacrylate ; les dérivés de benzotriazole ; les dérivés de benzalmalonate ; les dérivés de benzimidazole ; les imidazolines ; les dérivés bis-benzoazolyle tels que décrits dans les brevets EP669323 et US 2,463,264; les dérivés de l'acide p-aminobenzoïque (PABA) ; les dérivés de méthylène bis-(hydroxyphényl benzotriazole) tels que décrits dans les demandes US5,237,071, US5,166,355, GB2303549, DE 197 26 184 et EP893119 ; les polymères filtres et silicones filtres tels que ceux décrits notamment dans la demande WO-93/04665 ; les dimères dérivés d'α-alkylstyrène tels que ceux décrits dans la demande de brevet DE19855649 ; les 4,4-diarylbutadiènes tels que décrits dans les demandes EP0967200, DE19746654, DE19755649, EP-A-1008586, EP1133980 et EP133981 et leurs mélanges.

A titre d'illustration, comme d'agents photoprotecteurs actifs dans l'UV-A et/ou l'UV-B, on peut citer, désignés ci-dessous sous leur nom INCI :

les dérivés de l'acide p-aminobenzoïque (PABA), en particulier le PABA, l'éthyl PABA, l'éthyl Dihydroxypropyl PABA, l'éthylhexyl Diméthyl PABA (vendu notamment sous le nom « ESCALOL 507 » par ISP), le glyceryl PABA, ou le PEG-25 PABA (vendu sous le nom « UVINUL P25 » par BASF),

les dérivés salicyliques, en particulier l'homosalate (vendu sous le nom « EUSOLEX HMS » par RONA/EM INDUSTRIES), l'éthylhexyl salicylate (vendu sous le nom « NEO HELIOPAN OS » par HAARMANN et REIMER), le dipropyleneglycol salicylate (vendu sous le nom « DIPSAL » par SCHER), ou le TEA salicylate (vendu sous le nom « NEO HELIOPAN TS » par HAARMANN et REIMER),

les dérivés de dibenzoylméthane, en particulier le butyl Methoxydibenzoylmethane (vendu notamment sous le nom commercial « PARSOL 1789 » par HOFFMANN LA ROCHE), ou l'isopropyl Dibenzoylmethane,

les dérivés cinnamiques, en particulier l'éthylhexyl Methoxycinnamate (vendu notamment sous le nom commercial « PARSOL MCX » par HOFFMANN LA ROCHE), l'isopropyl methoxy cinnamate, l'isoamyl Methoxy cinnamate (vendu sous le nom commercial «NEO HELIOPAN E 1000 » par HAARMANN et REIMER), le cinoxate, le DEA methoxycinnamate, le diisopropyl methyl cinnamate, ou le glyceryl ethylhexanoate dimethoxycinnamate,

les dérivés de β,β-diphénylacrylate, en particulier l'octocrylene (vendu notamment sous le nom commercial « UVINUL N539 » par BASF), ou l'étocrylene, (vendue notamment sous le nom commercial « UVINUL N35 » par BASF),

les dérivés de la benzophénone, en particulier la benzophenone-1 (vendue sous le nom commercial « UVINUL 400 » par BASF), la benzophenone-2 (vendue sous le nom commercial « UVINUL D50 » par BASF), la benzophenone-3 ou oxybenzone (vendu sous le nom commercial « UVINUL M40 » par BASF), la benzophenone-4 (vendue sous le nom commercial « UVINUL MS40 » par BASF), la benzophenone-5, la benzophenone-6 (vendue sous le nom commercial « HELISORB 11 » par NORQUAY), la benzophenone-8 (vendue sous le nom commercial « SPECTRA-SORB UV-24 » PAR AMERICAN CYANAMID), la benzophenone-9 (vendue sous le nom commercial « UVINUL DS-49» par BASF), la benzophenone-12, ou le 2-(4-diéthylamino-2-hydroxybenzoyl)-benzoate de n-hexyle,

les dérivés du benzylidène camphre, en particulier le 3-benzylidene camphor (fabriqué sous le nom « MEXORYL SD» par CHIMEX), le 4-methylbenzylidene camphor (vendu sous le nom « EUSOLEX 6300 » par MERCK), le benzylidene camphor Sulfonic Acid (fabriqué sous le nom « MEXORYL SL» par CHIMEX), le camphor benzalkonium methosulfate (fabriqué sous le nom « MEXORYL SO » par CHIMEX), le terephthalylidene dicamphor sulfonic acid (fabriqué sous le nom « MEXORYL SX » par CHIMEX), ou le polyacrylamidomethyl benzylidene camphor (fabriqué sous le nom « MESORYL SW » par CHIMEX),

les dérivés de benzimidazole, en particulier le phenylbenzimidazole sulfonic acid (vendu notamment sous le nom commercial «EUSOLEX 232 » par MERCK), ou le disodium phenyl dibenzimidazole tetra-sulfonate (vendu sous le nom commercial « NEO HELIOPAN AP » par HAARMANN et REIMER),

les dérivés de triazine, en particulier l'anisotriazine (vendue sous le nom commercial «TINOSORB S » par CIBA SPECIALTY CHEMICALS), l'éthylhexyl triazone (vendue notamment sous le nom commercial «UVINUL T150 » par BASF), la diethylhexyl butamido triazone (vendue sous le nom commercial « UVASORB HEB » par SIGMA 3V), ou la 2,4,6- tris-(4'amino-benzalmalonate de diisobutyle)-s-triazine,

les dérivés de benzotriazole, en particulier, le drometrizole trisiloxane (vendu sous le nom « SILATRIZOLE » par RHODIA CHIMIE), le methylène bis-benzotriazolyl tetramethylbutylphénol (vendu sous forme solide sous le nom commercial « MIXXIM BB/100 » par FAIRMOUNT CHEMICAL ou sous forme micronisé en dispersion aqueuse sous le nom commercial « TINOSORB M » par CIBA SPECIALTY CHEMICALS),

les dérivés anthraniliques, en particulier le menthyl anthranilate (vendu sous le nom commercial « NEO HELIOPAN MA » par HAARMANN et REIMER),

les dérivés d'imidazolines, en particulier l'éthylhexyl dimethoxybenzylidene dioxoimidazoline propionate,

les dérivés de benzalmalonate, en particulier le polyorganosiloxane à fonctions benzalmalonate (vendu sous la dénomination commerciale « PARSOL SLX » par HOFFMANN LA ROCHE),

les dérivés de 4,4-diarylbutadiène, en particulier le 1,1'-dicarboxy (2,2'-diméthylpropyl)-4,4-diphénylbutadiène,

et leurs mélanges.

Les agents photoprotecteurs organiques plus particulièrement préférés sont choisis parmi l'éthylhexyl salicylate, l'éthylhexyl methoxycinnamate, l'octocrylene, le phenylbenzimidazole sulfonic acid, la benzophenone-3, la benzophenone-4, la benzophenone-5, la 4-methylbenzylidene camphor, le terephthalylidene dicamphor sulfonic acid, le disodium phenyl dibenzimidazole tetra-sulfonate, la 2,4,6-tris-(4'-amino benzalmalonate de diisobutyle)-s-triazine, l'anisotriazine, l'éthylhexyl triazone, la diethylhexyl butamido triazone, le methylène bis-benzotriazolyl tetramethylbutylphénol, le drometrizole trisiloxane, le 1,1'-dicarboxy (2,2'-diméthyl-propyl)-4,4-diphénylbutadiène, et leurs mélanges.

Les agents photoprotecteurs inorganiques sont choisis parmi des pigments ou bien encore des nanopigments (taille moyenne des particules primaires: généralement entre 5 nm et 100 nm, de préférence entre 10 nm et 50 nm) d'oxydes métalliques enrobés ou non comme par exemple des nanopigments d'oxyde de titane (amorphe ou cristallisé sous forme rutile et/ou anatase), de fer, de zinc, de zirconium ou de cérium qui sont tous des agents photoprotecteurs UV bien connus en soi. Des agents d'enrobage classiques sont par ailleurs l'alumine et/ou le stéarate d'aluminium. De tels nanopigments d'oxydes métalliques, enrobés ou non enrobés, sont en particulier décrits dans les demandes de brevets EP518772 et EP518773.

Les agents photoprotecteurs sont généralement présents dans les compositions selon l'invention dans des proportions allant de 0,1 à 20% en poids par rapport au poids total de la composition, et de préférence allant de 0,2 à 15% en poids par rapport au poids total de la composition.

Dans un autre aspect avantageux de l'invention, la composition utilisée peut contenir en outre au moins un autre actif agissant sur la fonction barrière de la peau, ou favorisant l'hydratation de la peau, et/ou un agent desquamant.

Par "agent desquamant", on entend tout composé capable d'agir :

- soit directement sur la desquamation en favorisant l'exfoliation, tel que les β-hydroxyacides, en particulier l'acide salicylique et ses dérivés (dont l'acide n-octanoyl 5-salicylique) ; les α-hydroxyacides, tels que les acides glycolique, citrique, lactique, tartrique, malique ou mandélique ; l'urée ; l'acide gentisique ; les oligofucoses ; l'acide cinnamique ; l'extrait de Saphora japonica ; le resvératrol ;

- soit sur les enzymes impliquées dans la desquamation ou la dégradation des cornéodesmosomes, telles que les glycosidases, la stratum corneum chymotryptic enzym (SCCE) voire d'autres protéases (trypsine, chymotrypsine-like). On peut citer les agents chélatant des sels minéraux : l'EDTA ; l'acide N-acyl-N,N',N' éthylène diaminetriacétique ; les composés aminosulfoniques et en particulier l'acide N-2-(hydroxyéthyl)piperazine-N'-2-éthane sulfonique (HEPES) ; les dérivés de l'acide 2-oxothiazolidine-4-carboxylique (procystéine) ; les dérivés d'acides alpha aminés de type glycine (tels que décrits dans EP-0 852 949, ainsi que le méthyl glycine diacétate de sodium commercialisé par BASF sous la dénomination commerciale TRILON M) ; le miel ; les dérivés de sucre tels que l'O-octanoyl-6-D-maltose et la N-acétyl glucosamine.

Parmi les actifs agissant sur la fonction barrière de la peau, ou favorisant l'hydratation de la peau, on peut citer :

- soit les composés agissant sur la fonction barrière, en vue de maintenir l'hydratation du stratum corneum, ou les composés occlusifs, en particulier les céramides, les composés à base sphingoïde, les lécithines, les glycosphingolipides, les phospholipides, le cholestérol et ses dérivés, les phytostérols (stigmastérol, $\beta$-sitostérol, campestérol), les acides gras essentiels, le 1-2 diacylglycérol, la 4-chromanone, les triterpènes pentacycliques tels que l'acide ursolique, la vaseline et la lanoline ;

- soit les composés augmentant directement la teneur en eau du stratum corneum, tel que le thréalose et ses dérivés, l'acide hyaluronique et ses dérivés, le glycérol, le pentanediol, le pidolate de sodium, la sérine, le xylitol, le lactate de sodium, le polyacrylate de glycérol, l'ectoïne et ses dérivés, le chitosane, les oligo- et polysaccharides, les carbonates cycliques, l'acide N-lauroyl pyrrolidone carboxylique, et la N-$\alpha$-benzoyl-L-arginine ;

- soit les composés activant les glandes sébacées tel que les dérivés stéroïdiens (dont la DHEA) et la vitamine D et ses dérivés.

La composition selon l'invention peut être appliquée sur la peau ou les muqueuses. Elle peut ainsi être utilisée dans un procédé de traitement cosmétique en vue de favoriser la synthèse des céramides, et/ou d'améliorer la fonction barrière de la peau ou des muqueuses, comprenant l'application de la composition selon l'invention sur la peau ou les muqueuses.

La présente invention concerne également un procédé de traitement cosmétique de la peau comprenant l'application de la composition selon l'invention sur la peau, en vue de maintenir l'éclat du teint et/ou de prévenir et/ou traiter la rugosité de la peau.

La présente invention concerne en outre un procédé cosmétique d'hydratation de la peau ou des muqueuses comprenant l'application d'une composition selon l'invention sur la peau ou les muqueuses.

Un autre aspect de l'invention concerne un procédé de traitement cosmétique des peaux sèches comprenant l'application d'une composition selon l'invention sur la peau.

En variante, la composition selon l'invention peut être utilisée pour la fabrication d'une préparation dermatologique comprenant une phase aqueuse, destinée à favoriser la synthèse des céramides, et/ou à améliorer la fonction barrière de la peau.

Les exemples qui suivent servent à illustrer l'invention sans toutefois présenter un caractère limitatif. Les composés sont, selon le cas, cités en noms chimiques ou en noms CTFA (International Cosmetic Ingredient Dictionary and Handbook).

**EXEMPLES**

**Exemple 1 : Test de conservation accéléré.**

Ce test a pour but d'étudier la dégradation d'un actif hydrophile sensible à l'oxydation après deux mois de conservation à 45°C. Diverses solutions ont été réalisées, et leurs compositions sont regroupées dans le tableau suivant :

Tableau I :

| Compositions (dans l'eau) | solution A (Témoin) | solution B | solution C | solution D |
|---|---|---|---|---|
| acide ascorbique | 15% | 15 % | 15% | 15% |
| polymère 1 | - | 1% | - | - |
| polymère 2 | - | - | 1% | - |

Tableau I : (suite)

| Compositions (dans l'eau) | solution A (Témoin) | solution B | solution C | solution D |
|---|---|---|---|---|
| polymère 3 | - | - | - | 1% |

Toutes les solutions sont ramenées à pH 6 avec KOH 8,9 mole/l

Les pourcentages des polymères sont donnés en matière active.

Polymère 1 : Copolymère vinylpyrrolidone-vinylimidazole (50/50) vendu sous la référence LUVITEC VPI 55K72W de la société BASF (Masse mol. moyenne en poids $1,2.10^6$).

Polymère 2 : Copolymère vinylpyrrolidone-vinylimidazole (50/50) vendu sous la référence LUVITEC VPI 55K18P de la société BASF (Masse mol. moyenne en poids 10000).

Polymère 3 : Polyvinylpyrrolidone vendu sous la référence KOLLIDON 12PF de la société BASF (Masse mol. moyenne en poids 3000).

Le taux de dégradation mesuré est donné par le rapport

$$(C_0 - C_{2mois})/C_0$$

avec $C_0$ concentration en acide ascorbique à t=0 et $C_{2mois}$ la concentration en acide ascorbique à t=2 mois, dans les conditions indiquées dans le tableau ci-dessus.

La concentration en acide ascorbique est déterminée par la technique HPLC (système LaChrom Merck). Les conditions analytiques sont les suivantes :

Colonne Lichrosphere100 RP18 (250 mm)
Eluant : tampon phosphate O,1 M, pH 2,1
Débit : 1 ml/min.
Détection à 257 nm

Dilution de l'échantillon tel que la concentrattion en acide ascorbique soit comprise entre 0,05 et 1 mg/ml.

Les résultats obtenus sont regroupés dans le tableau II suivant :

Tableau II :

| | Taux de dégradation après 2 mois à 45°C (en %) | |
|---|---|---|
| | sous air, flacon verre ambré | sous azote, flacon aluminium |
| **solution A** | 43 | 19,4 |
| **solution B** | 10,8 | 1 |
| **solution C** | 23,4 | 4,5 |
| **solution D** | 35,8 | 15,7 |

On constate d'après le tableau II que la stabilité de l'acide ascorbique est améliorée en présence des polymères 1 et 2 de l'invention, même en présence de l'oxygène de l'air, comparativement au témoin. On constate également que l'homopolymère de N-vinylpyrrolidone seul ne suffit pas à stabiliser de façon efficace la solution d'acide ascorbique.

Les polymères cités étant hydrophiles, il suffira de les ajouter à une solution aqueuse d'acide ascorbique pour stabiliser ce dernier.

**Exemple 2 : Effet de la composition selon l'invention sur la différenciation des kératinocytes**

Principe de la méthode :

L'étude consiste à évaluer l'effet de l'association de l'acide ascobique et d'un polymère ou copolymère selon l'invention sur la différenciation des kératinocytes, par mesure de l'activité transglutaminase (TGk) de kératinocytes épidermiques humains en culture. Le TGk est un marqueur de la différenciation terminale du kératinocyte et de la formation de l'enveloppe cornifiée (cornéocyte).

Des kératinocytes épidermiques humains utilisés au 3ème passage sont ensemencés en plaque 96 puits à la densité de 10.000 cellules par puits et cultivées en milieu SFM complet (Gibco 170005034, EGF et extrait pituitaire). Après 24 heures de pré-incubation, les cellules sont mises au contact du produit (testé à 30 µM) pendant 48 heures. Les cellules sont ensuite lavées puis soniquées sur glace en tampon Tris/EDTA, pH 8. L'enzyme membranaire est extraite en présence de TritonX100. L'activité TGk est dosée en mesurant l'addition covalente de putrescine tritiée à 2 µCi/ml final, à la caséine. La caséine est précipitée par l'acide trichloracétique à 20% contenant 1 mM de putrescine. Les précipités sont collectés sur filtres et collecteur Skatron. Les précipités sont lavés en milieu TCA 5% contenant 0,1 mM de putrescine et de l'éthanol. Les filtres secs sont comptés par scintillation liquide. Les protéines sont dosées sur chaque échantillon à l'aide d'un kit Dc Protein Assay (BioRad). Les activités TGk sont rapportées en µg de protéine.

Le rétinol ($10^{-6}$ M) et un milieu pauvre en calcium ont servi de références d'effet anti-différenciant.

Les comparaisons intergroupes ont été réalisées par analyse de variance (ANOVA) à l'aide du test de comparaison multiple de Dunnett.

Résultats :

Les résultats montrent que l'association de l'acide asciorbique et d'un copolymère N-vinylimidazole / N-vinylpyrrolidone provoque une augmentation significative de l'activité TGk présente dans les cultures de kératinocytes et donc une activité pro-différenciante vis-à-vis des kératinocytes. Par ailleurs, ce composé n'exerçait aucune cytotoxicité dans les conditions de l'essai.

**Exemple 3** : fluide H/E

On prépare la composition suivante de manière classique pour l'homme du métier.

| | |
|---|---|
| Methyl glucose sesquistearate | 2 g |
| Stearyl alcohol and ceteareth-20 | 2 g |
| Cyclohexasiloxane | 10 g |
| Propyl paraben | 0,1 g |
| Prunus armeniaca (apricot) kernel oil | 6 g |
| Shorea robusta seed butter | 2 g |

| | |
|---|---|
| Eau | 13,85 g |
| Methylparaben | 0,25 g |
| Peg-20 methyl glucose sesquistearate | 2 g |

| | |
|---|---|
| Ascorbic acid | 5 g |
| Potassium hydroxide (50% solution) | 2,97 g |
| Vinylpyrrolidone / vinylimidazole copolymer | 1 g |
| Eau | 41,03 g |

| Polyacrylamide and C13-14 isoparaffin and Laureth-7 | 1 g |
| Xanthan gum | 0,25 g |
| Chlorhexidine digluconate | 0,05 g |
| Eau | 10,5 g |

On obtient un fluide doux et frais qui améliore l'aspect de la peau grâce à une meilleure hydratation, et dans lequel l'acide ascorbique présente une bonne stabilité.

**Exemple 4 :** crème H/E

On prépare la composition suivante de manière classique pour l'homme du métier.

| **Phase A :** | |
| --- | --- |
| Glyceryl stearate (and) Peg-100 stearate | 2,1 g |
| Polysorbate 60 | 0,9 g |
| Cetyl alcohol | 2,6 g |
| Hydrogenated polyisobutene | 12 g |
| Cyclomethicone | 8 g |

| **Phase B**: | |
| --- | --- |
| Eau | 59,23 g |
| Glycerin | 2 g |
| Ascorbic acid | 5 g |
| Potassium hydroxide (50% solution) | 3,07 g |
| Vinylpyrrolidone/vinylimidazole copolymer | 1 g |
| Xanthan gum | 0,1 g |
| Carbomer | 0,4 g |

| **Phase C**: | |
| --- | --- |
| Triethanolamine | 0,3 g |
| Eau | 3 g |
| Conservateurs | 0,3 g |

On obtient un fluide doux et frais qui procure une meilleure hydratation et dans lequel l'acide ascorbique présente une bonne stabilité

**Revendications**

1. Utilisation cosmétique pour favoriser la synthèse des céramides et/ou améliorer la fonction barrière de la peau, d'une composition contenant, dans un milieu physiologiquement acceptable comprenant une phase aqueuse, au moins un actif hydrophile sensible à l'oxydation choisi parmi l'acide ascorbique et ses dérivés et au moins un polymère ou copolymère de N-vinylimidazole non réticulé, ledit actif et ledit polymère ou copolymère étant tous deux dans la phase aqueuse.

2. Utilisation cosmétique d'une composition contenant, dans un milieu physiologiquement acceptable comprenant une phase aqueuse, au moins un actif hydrophile sensible à l'oxydation choisi parmi l'acide ascorbique et ses dérivés et au moins un polymère ou copolymère de N-vinylimidazole non réticulé, ledit actif et ledit polymère étant tous deux dans la phase aqueuse, pour maintenir et/ou améliorer l'éclat du teint.

3. Utilisation cosmétique pour prévenir et/ou lutter contre la rugosité de la peau, d'une composition contenant, dans un milieu physiologiquement acceptable comprenant un phase aqueuse, au moins un actif hydrophile sensible à l'oxydation choisi parmi l'acide ascorbique et ses dérivés et au moins un polymère ou copolymère de N-vinylimidazole non réticulé, ledit actif et ledit polymère étant tous deux dans la phase aqueuse.

4. Utilisation d'au moins un actif hydrophile sensible à l'oxydation choisi parmi l'acide ascorbique et ses dérivés et d'au moins un polymère ou copolymère de N-vinylimidazole non réticulé pour la préparation d'une composition dermatologique comprenant une phase aqueuse, destinée à favoriser la synthèse des céramides et/ou à améliorer la fonction barrière de la peau.

5. Utilisation selon l'une quelconque des revendications 1 à 4, **caractérisée en ce que** l'actif hydrophile est choisi parmi les dérivés de l'acide ascorbique tels que le 5,6-di-O-diméthylsilyl-ascorbate, le dl-alpha-tocopheryl-dl-ascorbyl-phosphate sel de potassium, l'ascorbyl phosphate de magnésium, et l'ascorbyl phosphate de sodium.

6. Utilisation selon l'une quelconque des revendications 1 à 4, **caractérisée en ce que** l'actif hydrophile sensible à l'oxydation est l'acide ascorbique.

7. Utilisation selon l'une quelconque des revendications 1 à 6, **caractérisée en ce que** le copolymère non réticulé est une association de sous unités N-vinylimidazole avec des sous unités N-vinylpyrrolidone et/ou N-vinylcaprolactam.

8. Utilisation selon la revendication 7, **caractérisée en ce que** le copolymère non réticulé est un copolymère de N-vinylimidazole / N-vinylpyrrolidone.

9. Utilisation selon l'une quelconque des revendications 1 à 8, **caractérisée en ce que** le copolymère non réticulé est choisi parmi un copolymère vinylpyrrolidone/vinylimidazole (50/50) ayant une masse molaire moyenne en poids de 1 200 000 et un copolymère vinylpyrrolidone/vinylimidazole (50/50) ayant une masse molaire moyenne en poids de 10 000.

10. Utilisation selon l'une quelconque des revendications 1 à 9, **caractérisée en ce que** le rapport molaire entre l'équivalent unité N-vinylimidazole et l'actif hydrophile sensible à l'oxydation varie entre 0,004 et 16.

11. Utilisation selon la revendication 10, **caractérisée en ce que** le rapport molaire entre l'équivalent unité N-vinylimidazole et l'actif hydrophile sensible à l'oxydation varie entre 0,01 et 1.

12. Utilisation selon l'une quelconque des revendications 1 à 11, **caractérisée en ce que** le polymère ou copolymère est présent à une concentration comprise entre 0,1 et 5 % en poids de la phase aqueuse.

13. Utilisation selon la revendication 12, **caractérisée en ce que** le polymère ou copolymère est présent à une concentration comprise entre 0,1 et 2 % en poids de la phase aqueuse.

14. Utilisation selon l'une quelconque des revendications 1 à 13 **caractérisée en ce que** le polymère ou copolymère possède une fraction molaire en unité N-vinylimidazole comprise entre 0,1 et 1.

15. Utilisation selon la revendication 14, **caractérisée en ce que** le polymère ou copolymère possède une fraction molaire en unité N-vinylimidazole comprise entre 0,4 et 0,9.

16. Utilisation selon l'une quelconque des revendications 1 à 15, **caractérisée en ce que** la composition contient en outre un autre agent agissant sur la fonction barrière de la peau, ou favorisant l'hydratation de la peau et/ou un agent desquamant .

17. Utilisation selon la revendication 16, **caractérisée en ce que** l'agent agissant sur la fonction barrière de la peau, ou favorisant l'hydratation de la peau est choisi parmi les céramides, les composés à base sphingoïde, les lécithines, les glycosphingolipides, les phospholipides, le cholestérol et ses dérivés, les phytostérols (stigmastérol, β-sitostérol, campestérol), les acides gras essentiels, le 1-2 diacylglycérol, la 4-chromanone, les triterpènes pentacycliques tels que l'acide ursolique, la vaseline, la lanoline, le thréalose et ses dérivés, l'acide hyaluronique et ses dérivés, le glycérol, le pentanediol, le pidolate de sodium, la sérine, le xylitol, le lactate de sodium, le polyacrylate de glycérol, l'ectoïne et ses dérivés, le chitosane, les oligo- et polysaccharides, les carbonates cycliques, l'acide N-lauroyl pyrrolidone carboxylique, la N-α-benzoyl-L-arginine, la DHEA et les dérivés stéroïdiens, et la vitamine D et ses dérivés.

18. Utilisation selon la revendication 16, **caractérisée en ce que** l'agent desquamant est choisi parmi les β-hydroxyacides, les α-hydroxyacides, l'urée ; l'acide gentisique ; les oligofucoses ; l'acide cinnamique ; l'extrait de Saphora japonica ; le resvératrol, l'EDTA ; l'acide N-acyl-N,N',N' éthylène diaminetriacétique ; les composés aminosulfoniques, les dérivés de l'acide 2-oxothiazolidine-4-carboxylique (procystéine) ; les dérivés d'acides alpha aminés de type glycine; le miel ; les dérivés de sucre.

19. Procédé de traitement cosmétique destiné à maintenir et/ou améliorer l'éclat du teint, comprenant l'application sur la peau d'une composition contenant, dans un milieu physiologiquement acceptable, au moins un actif hydrophile sensible à l'oxydation choisi parmi l'acide ascorbique et ses dérivés et au moins un polymère ou copolymère de N-vinylimidazole non réticulé, ledit actif et ledit polymère étant tous deux dans la phase aqueuse.

20. Procédé cosmétique d'hydratation de la peau comprenant l'application sur la peau d'une composition contenant, dans un milieu physiologiquement acceptable, au moins un actif hydrophile sensible à l'oxydation choisi parmi l'acide ascorbique et ses dérivés et au moins un polymère ou copolymère de N-vinylimidazole non réticulé, ledit actif et ledit polymère étant tous deux dans la phase aqueuse.

21. Procédé cosmétique de traitement des peaux sèches, comprenant l'application sur la peau d'une composition contenant, dans un milieu physiologiquement acceptable, au moins un actif hydrophile sensible à l'oxydation choisi parmi l'acide ascorbique et ses dérivés et au moins un polymère ou copolymère de N-vinylimidazole non réticulé, ledit actif et ledit polymère étant tous deux dans la phase aqueuse.

# EP 1 316 304 A1

**Office européen des brevets**

## RAPPORT DE RECHERCHE EUROPEENNE

Numéro de la demande

EP 02 29 2810

### DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| A | EP 1 064 924 A (BASF AG) 3 janvier 2001 (2001-01-03) * revendication 1; exemple 10 * ----- | 1 | A61K7/48 |

|  | DOMAINES TECHNIQUES RECHERCHES (Int.Cl.7) |
|---|---|
|  | A61K A61Q |

Le présent rapport a été établi pour toutes les revendications

| Lieu de la recherche | Date d'achèvement de la recherche | Examinateur |
|---|---|---|
| LA HAYE | 3 février 2003 | Glikman, J-F |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

...............................................................................
& : membre de la même famille, document correspondant

EPO FORM 1503 03.82 (P04C02)

14

**ANNEXE AU RAPPORT DE RECHERCHE EUROPEENNE
RELATIF A LA DEMANDE DE BREVET EUROPEEN NO.**

EP 02 29 2810

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

03-02-2003

| Document brevet cité au rapport de recherche | Date de publication | Membre(s) de la famille de brevet(s) | | Date de publication |
|---|---|---|---|---|
| EP 1064924 A | 03-01-2001 | DE | 19929758 A1 | 04-01-2001 |
| | | BR | 0002906 A | 30-01-2001 |
| | | CN | 1282571 A | 07-02-2001 |
| | | EP | 1064924 A2 | 03-01-2001 |
| | | JP | 2001055321 A | 27-02-2001 |

EPO FORM P0460

Pour tout renseignement concernant cette annexe : voir Journal Officiel de l'Office européen des brevets, No.12/82